# EUROPEAN PATENT SPECIFICATION

(11) **EP 4 044 915 B1**
(45) Date of publication and mention of the grant of the patent: **27.12.2023**
(21) Application number: 20796721.7
(22) Date of filing: 15.10.2020
(51) Int. Cl.: A61B 5/06, A61B 34/20

(54) **AUTOMATIC MEDICAL INSTRUMENT IDENTIFICATION**
AUTOMATISCHE IDENTIFIZIERUNG EINES MEDIZINISCHEN INSTRUMENTS
IDENTIFICATION AUTOMATIQUE D'INSTRUMENT MÉDICAL

(30) Priority: 15.10.2019 US 201962915197 P
(43) Date of publication of application: 24.08.2022
(73) Proprietor: Fiagon GmbH, 16761 Hennigsdorf (DE)
(72) Inventor: NORMAN, Nicholas, Charlotte, North Carolina 28203 (US); HEDERMANN, Robert, 10409 Berlin (DE); BERGFELD, Malte, 12047 Berlin (DE); MUCHA, Dirk, 16548 Glienicke (DE); DESINGER, Kai, 10711 Berlin (DE)
(74) Representative: Eisenführ Speiser
(86) International application number: PCT/EP2020/079104
(87) International publication number: WO 2021/074326

(56) References cited:
- US-A1- 2003 066 538
- US-A1- 2004 024 309
- US-A1- 2014 303 489
- US-A1- 2017 196 508
- US-A1- 2019 038 366
- US-A2- 2018 021 092

## Description

### TECHNICAL FIELD

The invention relates to a medical instrument identification setup for automatically identifying a medical instrument. The invention also relates to a method for automatically identifying a medical instrument.

### BACKGROUND OF THE INVENTION

For assisting a surgeon in using a medical instrument in a surgical procedure it is known to track the position of the medical instrument inside a patient's body and to display the medical instrument's position in, e.g., sectional images of a model of a patient on a monitor.

To this end, surgical navigation systems are used typically comprising a position detection system, a monitor, and one or more localizers. The position detection system can be, e.g., an optical position detection system, an ultrasound-based position detection system or an electromagnetic position detection system. A position detection system, in general, is configured for determining position and orientation of localizers. The localizers can be mounted on a medical instrument to allow tracking of the medical instrument by means of the position detection system.

By way of example, electromagnetic position detection systems are known having a field generator for generating an alternating electromagnetic field. A medical instrument to be used with an electromagnetic position detection system is equipped with a localizer that typically comprises one or more sensor coils.

When exposed to an alternating electromagnetic field, in the sensor coils of a localizer a voltage is induced that depends on the position and orientation of a respective sensor coil in the alternating electromagnetic field. With a position detection system a sensor signal representing the induced voltage can be tapped from the sensor coils and analysed for determining position and orientation of the localizer. Typically, position and orientation of a localizer of a medical instrument are determined relative to the position and orientation of a reference localizer, sometimes called patient localizer, that can likewise comprise sensor coils and that stays fixed relative to a patient.

In order to calculate position and orientation of the medical instrument equipped with a localizer relative to a position detection system, often, it is required to calibrate the position of the medical instrument's tip to the position of the localizer. To this end, typically a calibration device is employed that has known position and orientation relative to the position detection system. For example, the calibration device can likewise be equipped with a localizer the position and orientation of which can be determined with the position detection system. By means of calibration, a transformation function, sometimes called calibration matrix, can be established representing the spatial relationship between the position and orientation of a medical instrument's localizer and the medical instrument's tip. The established transformation function can be used during a navigated procedure for displaying the position of the medical instrument's tip in sectional images of a patient's model on a monitor.

For displaying the medical instrument's position in sectional images of a patient's model on a monitor of a navigation system, it is typically further required to register the patient model to the patient. Often, a model of a patient is a topographic image that is generated from two-, three- or four-dimensional images of a patient obtained preoperatively by tomography, e.g., via computed tomography (CT), magnetic resonance imaging (MRI) or C-arm fluoroscopic imaging. Registration, in general, refers to obtaining the spatial correlation between position and orientation of a patient in real space (sometimes also called patient space) and the patient model, initially defined in terms of coordinates in the coordinate system of the respective two-, three- or four-dimensional image used for generating the patient model.

Having calibrated the medical instrument and having registered the patient model, the position of a medical instrument can be displayed in sectional images of the model for visually assisting a surgeon in navigating the medical instrument.

US 2018/0021092 A2 states in its abstract that it describes a data processing method for identifying a medical instrument which is configured to be tracked by means of a medical tracking system, wherein the method is designed to be executed by a computer and comprises acquiring relative position and/or movement data which comprise relative position and/or movement information describing a relative position and/or movement between a calibration tool and the medical instrument which is in contact with the side or edge of the at least one supporting surface.

US 2004/0024309 A1 states in its abstract that it describes a for monitoring the position of a medical instrument with respect to a patient's body and for displaying at least one of a plurality of prerecorded images of said body responsive to the position of said medical instrument.

US 2003/0066538 A1 states in its abstract that it describes an integrated surgical anchor/localization sensor is disclosed, wherein he anchor is adapted to be secured to an anatomical structure and contains a sensor housing.

US 2019/0038366 A1 states in its abstract that it describes devices, systems, and methods for determining the shape of a surgical implant and navigating the surgical implant during surgery.

US 2017/0196508 A1 states in its abstract that it provides a spinal device/implants, comprising a spinal device/implant and a sensor.

### SUMMARY OF THE INVENTION

It is an object of the invention to provide a medical instrument identification setup by means of which a medical instrument can automatically be identified. It is also an object of the invention to provide a method for automatically identifying a medical instrument.

According to the invention, a medical instrument identification setup is proposed comprising a sensor carrier equipped with at least localizers, a position detection system for determining position and orientation of the localizers, a calibration device serving as a calibration reference, a calibration unit for calibrating a medical instrument equipped with the sensor carrier, and a medical instrument identification unit for identifying the medical instrument.

In particular, the sensor carrier is configured to be removably arranged in a lumen of a medical instrument. The sensor carrier has at least two localizers, the localizers each being configured for providing a sensor signal representing position and orientation of the respective localizer. A medical instrument being equipped with the sensor carrier can be connected to the position detection system. With the sensor carrier being arranged in a medical instrument's lumen, position and orientation of the sensor carrier's localizers can be determined by the position detection system from provided sensor signals. From determined position and orientation of the sensor carrier's localizers, position and orientation of the medical instrument can be calculated by the position detection system.

Preferably, a first one of the sensor carrier's localizers is arranged at or at least close to the distal end of the sensor carrier, i.e., at a distance of 5 mm or less from the sensor carrier's distal end, and a second one of the localizers is arranged at a distance from the first localizer towards the proximal end of the sensor carrier.

Preferably, at least the sensor carrier's first localizer and a second localizer, each are configured to act as a 5 DOF sensor. Preferably, also in combination, the at least two localizers are configured to act as 5 DOF sensors. A 5 DOF senor can be realized by employing sensor coils.

If a localizer comprises one or more sensor coils that are exposed to an alternating electromagnetic field, a sensor signal can be tapped from each of the sensor coils, the sensor signal representing position and orientation of the respective sensor coil in an electromagnetic field. The tapped sensor signal can be transmitted via a wired connection or via a wireless connection to the position detection system.

The medical instrument identification setup's position detection system is configured for determining position and orientation of the at least two localizers in the position detection system's coordinate system from received sensor signals. Preferably, the position detection system is an electromagnetic position detection system comprising a field generator for generating an alternating electromagnetic field and a signal processing unit for processing sensor signals. Position and orientation of the at least two localizers each having one or more sensor coils can be determined in the alternating electromagnetic field from tapped sensor signals representing voltages induced in the sensor coils by the electromagnetic field. A position detection system's signal processing unit typically comprises an analog-to-digital converter and a digital signal processor.

For connecting a medical instrument to an electromagnetic position detection system, at least one of the sensor carrier's localizers, preferably, comprises at least one sensor coil. With one sensor coil only five degrees of freedom (DOF) can be determined. The sixth degree, namely, the rotation around the longitudinal axis of the sensor coil, cannot be determined. A 6 DOF sensor can be implemented in that the sensor carrier's localizer comprises at least two sensor coils that are arranged at an angle, e.g., orthogonally, to each other. A 6 DOF sensor can also be implemented in that the sensor carrier has two localizers each having a sensor coil, wherein the sensor coils of the localizers are arranged such that their longitudinal axis enclose a non-zero angle, preferably an angle of 90°.

The sensor carrier can also comprise at least a third localizer that is configured for providing a sensor signal representing position and orientation of the third localizer. Preferably, the third localizer is arranged at a distance from the first and second localizers towards the sensor carrier's proximal end. Preferably, the third localizer is arranged at or at least close to the sensor carrier's proximal end.

The at least two localizers alone or in combination with at least a third localizer can be used for bend detection as well. If the sensor carrier is inserted into the lumen of a rigid medical instrument and in the course of insertion, a change in the orientation and/or position of the localizers with respect to their relative positions to each other can be detected, the detected change can be used to determine a bend, i.e., an angle, in the medical instrument. Preferably, if a change in the orientation and/or position of the localizers is detected, e.g., with a position detection system, a warning signal is displayed on a monitor by means of the position detection system.

In particular, if the sensor carrier is arranged in a lumen of a medical instrument that has at least one angle along the length of its lumen, by means of determined position and orientation of the three localizers, it is possible to calculate the medical instrument's angle. Often, the determined angle is characteristic for a specific medical instrument and can be used for identifying the medical instrument with arranged sensor carrier with the medical instrument identification unit. For example, if the sensor carrier with at least three localizers is arranged in the lumen of an endoscope, preferably, the third localizer is located in an angled access port. Typically, the angle between access port and the rest of the endoscope is characteristic for the endoscope itself. From determined position and orientation of the third localizer and at least one of the at least two localizers, respectively, the endoscope's angle can be calculated by the position detection system and/or the calibration unit and used by the medical instrument identification unit for identifying the endoscope.

In case the sensor carrier comprises a third localizer, the third localizer, preferably, is arranged at or at least close to the sensor carrier's proximal end. In case the sensor carrier having a third sensor carrier arranged at or close to its proximal end, position and orientation of the third localizer, preferably, are used by the medical instrument identification unit for identifying the medical instrument and/or by the position detection system for calculating the sixth degree of freedom of the at least two localizers arranged within the medical instrument's distal end region. In case the localizers comprise sensor coils, calculating the sixth degree of freedom of the at least two localizers arranged within the medical instrument's distal end region can be achieved with the position detection system, in particular, when the third localizer's longitudinal axis has an angle to the longitudinal axis of the at least two localizers, respectively.

In order to be arranged in lumens of different medical instruments used during a navigated procedure, the distance between the sensor carrier's proximal end and the distal end is preferably between 10 cm and 200 cm, in particular, between 15 cm and 150 cm, more preferably, between 20 cm and 100 cm. The outer diameter of the sensor carrier is preferably equal to or smaller than 3 mm, in particular, equal to or smaller than 1.5 mm, more preferably, equal to or smaller than 1 mm.

Preferably, the sensor carrier comprises a hypo tube extending from the distal end to the proximal end of the sensor carrier and enclosing the sensor carrier's at least two localizers. Preferably, a hypo tube enclosing the at least two localizers is configured to give mechanical stability to the sensor carrier and to protect the localizers from external influences.

Suitable materials of which the hypo tube of the sensor carrier can be made are for example polyurethane (PUR), polyethylene, silicone rubber or polyether ether ketone (PEEK), nitinol, nitinol alloy or stainless steel. Typically, a hypo tube is a long metal tube with micro-engineered features along its length that shall provide the desired mechanical properties of the hypo tube. The sensor carrier with a hypo tube made of one of these materials can advantageously be repeatedly sterilized and is biocompatible.

The sensor carrier can be used for connecting generally known medical instruments having a lumen, e.g., a lumen that originally was provided for a guidewire, to a position detection system. To this end, the sensor carrier is inserted into the medical instrument's lumen. With the arranged sensor carrier, the medical instrument can be connected to and used with a position detection system in a navigated procedure. In particular, during a navigated procedure, the position of the medical instrument relative to a position detection system can be calculated from determined position and orientation of the at least two localizers with the position detection system. A calculated position of a medical instrument can be displayed in sectional images of a patient model on a monitor connected to the position detection system for assisting a surgeon in guiding the medical instrument inside a patient's body. After having accomplished a navigated procedure, the sensor carrier can be removed from the medical instrument's lumen and the lumen can be used as a working channel for other medical instruments such as drills or forceps.

Medical instruments that generally have a lumen in which the sensor carrier can be arranged for connecting the medical instrument to a position detection system are, e.g., catheters, biopsy needles, access needles, and cannulated instruments, e.g., a Jamshidi needle, taps, screwdrivers and bone screws, e.g., pedicle screws, that can be placed with the screwdriver. Such medical instruments having the sensor carrier arranged in their respective lumen can be automatically identified using the medical instrument identification setup.

Further medical instruments that can be equipped with the sensor carrier and, thus, automatically identified using the medical instrument identification setup are medical instruments that commonly do not have a lumen, but that have been retrofitted with a lumen or that are manufactured with a lumen suitable for insertion of the sensor carrier ab initio. Such a medical instrument can be, e.g., a scalpel, a surgical saw, e.g. a bone saw, a bone file, a cautery, or forceps.

The at least two localizers at the distal end of the sensor carrier can be configured to implement and/or can be treated as one sensor or as independent sensors. In case of the sensor carrier being arranged in the medical instrument's lumen, the position of the first localizer and the position of the second localizer along the longitudinal axis of the medical instrument can be used to calculate one virtual longitudinal axis between the two localizers by drawing a virtual line connecting the two central positions of the localizers, for creating a comparatively stable and accurate virtual axis for the sensor carrier and medical instrument.

In case the sensor carrier is arranged in a lumen of a medical instrument it is of advantage that the medical instrument's virtual longitudinal axis can be determined by the position detection system and/or the calibration unit based on position and/or orientation of the sensor carrier's at least two localizers. A medical instrument's virtual longitudinal axis, preferably, is the axis that intersects the positions of the at least two localizers of the arranged sensor carrier. The medical instrument's virtual longitudinal axis is defined in the coordinate system of the position detection system based on position and/or orientation of localizers. The physical instrument axis is defined by means of coordinates in real space.

Advantageously, by using the positions of at least two localizers for determining a medical instrument's virtual longitudinal axis, it is possible to reduce an angle error resulting, inter alia, from a physical misalignment of the sensor carrier inside a medical instrument's lumen to less than one degree. Advantageously, this allows for maintaining a stable central axis, e.g., during a navigated procedure, also when the medical instrument is rotated.

In general, it is not required to use the orientations (and thus only the position) of the sensor carrier's at least two localizers for determining the medical instrument's virtual longitudinal axis. However, the calibration unit and/or the position detection system can be configured to also use the orientations of the at least two localizers for determining the virtual longitudinal axis in addition to or instead of the localizers' positions.

By means of position and/or orientation of the at least two localizers, a spatial relationship between a medical instrument's virtual longitudinal axis and the medical instrument's physical longitudinal axis can be determined by the position detection system and/or the calibration unit. Thereby it is possible to define and ensure the trueness of the medical instrument's virtual longitudinal axis to the medical instrument's physical longitudinal axis based on position and/or orientation of the localizers, respectively.

In the sensor carrier, the at least two localizers are preferably arranged at a distance from each other that is chosen in dependence on the geometry of specific medical instruments of the surgical kit. Preferably, the distance between a sensor carrier's first localizer and a second localizer of the at least two localizers is between 25 cm and 5 cm, in particular, between 20 cm and 5 cm, even more preferably between 15 cm and 5 cm, e.g., 10 cm. A larger distance makes the determination of a medical instrument's virtual longitudinal axis more precise. Preferably, the at least two localizers are arranged within a distal end region of the sensor carrier, the distal end region extending from the sensor carrier's distal end up to the proximal end of that localizer of the at least two localizers, that is arranged closer towards the sensor carrier's proximal end. In other words, the distal end region preferably comprises that section of the sensor carrier that extends between the sensor carrier's distal end and the proximal end of that localizer of the at least two localizers that is arranged closer to the proximal end.

Preferably, the calibration unit and/or the position detection system are configured for extrapolating a medical instrument's virtual longitudinal axis from the distal end of the arranged sensor carrier to thus determine the position of a distal end of a medical instrument in the lumen of which the sensor carrier is arranged. The calibration unit can be configured for calibrating a medical instrument using the position of a distal end of a medical instrument as determined via extrapolation. The position detection system can be configured for visualizing the position of a medical instrument's tip in sectional images on a monitor using the extrapolated position of the medical instrument.

Besides automatic instrument identification, with a position detection system a medical instrument's virtual longitudinal axis can be calculated and displayed in sectional images of a patient model on a monitor to visualize position and orientation of the medical instrument with respect to patient's body part. The position detection system can be connected to the medical instrument identification unit to obtain which medical instrument has been identified. The position detection system can be configured for automatically adapting the settings for displaying a digital representation of the medical instrument on a connected monitor for the identified medical instrument.

Displaying a medical instrument's virtual longitudinal axis with respect to a patient's body part can be of particular relevance during a navigated procedure, e.g., when aiming at an anatomical target from a comparatively large distance, e.g., a distance of 5 cm to 15 cm, with a medical instrument, e.g., an access needle, with arranged sensor carrier.

The position detection system can be configured for comparing determined position and orientation of the at least two localizers and for determining distortions of a navigation field, e.g., of an electromagnetic field, based on the comparison. For example, electromagnetic field distortions can be caused by a metal object located close to the sensor coils of one of the localizers. Due to electromagnetic field distortions, position and orientation of a respective localizer cannot be determined correctly, thus, resulting in an incorrect calculation of the position of the medical instrument's tip by means of the position detection system. If electromagnetic field distortions are detected, the position detection system can be configured for performing a plausibility check for identifying a localizer the position and orientation of which can currently be reliably determined. For example, the position detection system can be configured for comparing the current position and orientation of a localizer with previously determined position and orientation of the localizer for checking whether position and orientation of this localizer can currently be reliably determined.

The medical instrument identification setup's calibration device is configured such that its position and orientation in the position detection system's coordinate system can be determined by the position detection system. For example, the calibration device can be arranged at a position whose coordinate is known in the position detection system's coordinate system. It is also possible that the calibration device likewise is equipped with at least one localizer that is configured to provide a sensor signal representing the localizer's position and orientation.

The calibration device can have different calibration regions that are advantageously designed for the calibration of different medical instruments, e.g., adapted to the medical instrument's geometries. Different calibration regions can be implemented by visual indicators on the calibration device. The calibration unit can be configured for detecting which of the different calibration regions has been contacted with a medical instrument's tip. The medical instrument identification unit can be configured for identifying a medical instrument based on which of the different calibration regions has been contacted with a medical instrument's tip.

Advantageously, with different calibration regions on the calibration device that, preferably, are visual indicators on the calibration device, it allows for automatically differentiating between two medical instruments, also in case the two respective medical instruments have the same working length.

The calibration unit can be configured to calculate a centering error and/or a positioning error and/or an angle error and to compensate for the calculated centering error and/or positioning error and/or angle error when calculating the distance between the calibration device and at least one of the two localizers.

The calibration device can be complemented by and thus be linked to a specific procedure and/or surgical workflow in the software of the medical instrument identification setup to enable automatic instrument identification by means of the procedure steps. If the calibration device is linked to a specific software workflow, a medical instrument can be calibrated on the calibration device for identifying the medical instrument and for displaying the corresponding visualization and/or workflow.

The linking of the calibration device to specific procedure and/or surgical workflow in the software can be implemented to enable automatically differentiating between two medical instruments that have the same working length. The software algorithm implementing the medical instrument identification can be configured for recognizing the possible medical instruments resulting from the calibration procedure and for proposing the medical instrument that is used in the current step of the workflow.

Preferably, the algorithm is configured to visualize all identified medical instruments which result from a calibration procedure and highlights that medical instrument which is proposed according to the predefined workflow for confirmation to a user. A user can choose for this medical instrument, but also for any other medical instrument in case the user did not follow the workflow.

Most preferred, the algorithm is configured to display the proposed medical instrument and other possible instruments for selection by a user for, e.g., a 3 second, 5 second or 10 second time duration, while, preferably, also displaying the remaining time, e.g., as a count down. The algorithm can be configured to automatically select the proposed medical instrument, if a user does not do any selection.

The medical instrument identification setup's calibration unit is configured for calibrating a medical instrument with the sensor carrier arranged in its lumen. Calibrating a medical instrument with the sensor carrier comprises calculating a distance between the medical instrument's tip and at least one of the two localizers based on the position and orientation of the calibration device and the position and orientation of at least one of the two localizers as determined by the position detection system. For example, for calibrating the medical instrument with the sensor carrier arranged in the medical instrument's lumen, the medical instrument's tip can be brought into contact with the calibration device.

The calibration unit can be configured for automatically detecting whether or not the medical instrument's tip is in contact with the calibration device by determining whether a virtual longitudinal axis defined by the positions of the at least two localizers intersects the known position of a calibration device over a predefined period of time and/or whether the distance between the calibration device and at least one of the localizers stays constant over a predefined period of time.

The calibration unit can be configured to start automatic calibration of the medical instrument when the medical instrument's tip is found to be in contact with the calibration device. In particular, when in contact, the distance between the calibration device and at least one of the two localizers can be determined which corresponds to the distance between the medical instrument's tip and the respective one of the at least two localizers.

By means of calibration, a transformation function can be established by the calibration unit, the transformation function representing the spatial relationship between the medical instrument's tip and the at least two localizers.

The medical instrument identification setup's medical instrument identification unit is configured for determining a length of the medical instrument's lumen at least from the calculated distance and for using the determined length of the lumen for identifying the medical instrument. In particular, the medical instrument identification unit is connected to the calibration unit for obtaining the calculated distance between the medical instrument's tip and the at least two localizers.

The invention includes the recognition that the length of a lumen of a medical instrument, typically, is characteristic for the medical instrument itself. Thus, a medical instrument can be identified by virtue of the length of its lumen.

The invention includes the further recognition that the length of a lumen of a medical instrument can be automatically obtained when calibrating the medical instrument with sensor carrier being arranged in the medical instrument's lumen. Thus, by means of the sensor carrier, the length of the medical instrument's lumen can automatically be determined and used for identifying the medical instrument.

Automatically obtaining the length of a medical instrument's lumen can be achieved with the medical instrument identification setup in that with the calibration unit the distance between the medical instrument's tip and at least one of the two localizers is calculated and in that the calculated distance is used by the medical instrument identification unit for determining the length of the medical instrument's lumen. The medical instrument identification unit is configured for automatically identifying the medical instrument based on the determined length of the lumen.

As a result, it is possible to automatically identify a calibrated medical instrument with arranged sensor carrier by means of the medical instrument identification setup's a medical instrument identification unit based on the length of the medical instrument's lumen.

The information of the identified medical instrument can be used, e.g., for adapting the way of displaying or visualizing the medical instrument on a monitor. For example, to visualize on a monitor a digital representation of that medical instrument that has been identified by a medical instrument identification unit, i.e., the medical instrument representation in 2D or 3D views can be specifically accurate to the identified medical instrument.

It is also possible to use information of the identified medical instrument for automatically adapting a workflow in software according to how the identified medical instrument is used in a navigated procedure.

It is also possible to use information of the identified medical instrument for automatically adapting the views on a monitor for the instrument that is being used. For example, if an endoscope is identified during a navigated procedure, the software can automatically switch to displaying the video view from the endoscope in parallel with navigation views. The medical instrument identification can also be linked to a defined software procedure workflow, where the combination of the medical instrument identification and software workflow can guide a surgeon through a navigated procedure.

Medical instruments that can be identified with the medical instrument identification setup comprise medical instruments that generally have a lumen in which the sensor carrier can be arranged for operatively connecting the medical instrument to a position detection system. Such medical instruments that generally have a lumen are, e.g., catheters, access needles, taps, screwdrivers and bone screws, e.g., pedicle screws, that can be placed with the screwdriver.

Further medical instruments that can be equipped with the sensor carrier are medical instruments that commonly do not have a lumen, but that have been retrofitted with a lumen or that are manufactured with a lumen suitable for insertion of the sensor carrier ab initio. Such a medical instrument can be a scalpel, a surgical saw, e.g. a bone saw, a bone file, a cautery, forceps, an endplate rasp, a cage trial device, or a cage placement device.

A medical instrument can be identified at least based on the distance between the medical instrument's tip and at least one of the two localizers which can be determined based on the position and orientation of the calibration device and the position and orientation of at least one of the two localizers. Additionally to the calculated distance, a medical instrument can be identified based on a calculated angle of a medical instrument and/or the length of that section of the sensor carrier that lies outside a medical instrument's lumen in which the sensor carrier is arranged, i.e., that part of the senor carrier that extends beyond the proximal end of the medical instrument's lumen.

By way of example, it is possible to automatically determine the length of the medical instrument's lumen based on the sensor carrier having a fixed length and the localizers of the sensor carrier being located at fixed relative positions, e.g., within a hypo tube. Preferably, the length of the sensor carrier is shorter than the length of the lumens of those medical instruments, that shall potentially be automatically identified during a navigated procedure. For example, the sensor carrier can have a length that corresponds to the length of the lumen of that medical instrument out of a plurality of medical instruments that shall be potentially be automatically identified that has the lumen with shortest length. Thus, when successively inserting the sensor carrier in each of the number of medical instruments that shall potentially be automatically identified during a navigated procedure, for each of the medical instruments the distance between its tip and the localizers of the arranged sensor carrier is different compared to the distances obtained for other medical instruments out of the number of medical instruments that shall potentially be automatically identified. For example, it is possible to first calculate the distance between a medical instrument's tip and at least one of the sensor carrier's localizers and to add the remaining length of the sensor carrier to the calculated distance in order to obtain the length of the medical instrument's lumen. For example, the value of the length of the sensor carrier can be provided a priori to a medical instrument identification setup's medical instrument identification unit and the medical instrument identification unit can be configured for using the provided sensor carrier length for determining the length of a medical instrument's lumen. In particular, if the position of a sensor carrier's localizer relative to the sensor carrier's distal end and/or proximal end is known, the distance from the localizer to the sensor carrier's distal end and/or proximal end, respectively, can be used for determining the length of a medical instrument's lumen accommodating the sensor carrier.

If the sensor carrier is arranged in a medical instrument's lumen, the distance between the sensor carrier's localizers and the medical instrument's tip is characteristic for the length of the medical instrument's lumen. Therefore, from the calculated distance between localizers and the medical instrument's tip, the length of the lumen itself can be derived.

The medical instrument identification setup's sensor carrier can be provided with its longitudinal axis being pre-calibrated in the position detection system's coordinate system. A calibration matrix representing the spatial relationship can be used for calibrating and navigating a medical instrument having the sensor carrier arranged in its lumen.

In particular, if the medical instrument has a straight lumen, the sensor carrier's longitudinal axis matches the medical instrument's physical axis. The calibration matrix of the pre-calibrated medical instrument can advantageously be used by the position detection system and/or the calibration unit for calculating the medical instrument's virtual longitudinal axis and for extrapolating the medical instrument's distal end.

It is advantageous if the sensor carrier itself is rigid at least in that section in which the localizers are arranged such that the pre-calibrated state remains valid also outside the straight lumen of a medical instrument.

The medical instrument identification setup according to the invention can be used together with a surgical kit as part of a medical system, the surgical kit comprising a plurality of medical instruments having a lumen in which at least a part of the sensor carrier can be arranged. The medical instrument identification setup according to the invention is particularly suitable for identifying medical instruments of a surgical kit that each have a lumen with a length that is different to the lengths of the lumens of the other medical instruments of the surgical kit.

Preferably, for identifying a medical instrument of the surgical kit, a sensor carrier can be used that has a length that is equal to or smaller than the length of the lumen of that medical instrument of the surgical kit that has the shortest lumen. In this case, the sensor carrier can be arranged in each of the medical instrument's lumen with its full length. For each of the medical instruments of the surgical kit, the distance between the respective medical instrument's tip and the sensor carrier's localizers is unique and can be used for differentiating the medical instruments from others of the surgical kit. A distance calculated for one of the medical instruments can thus be used for identifying the medical instrument.

For identifying a medical instrument of the surgical kit, it is also possible to use a sensor carrier that has a length that is larger than the lumens of at least two of the surgical kit's medical instruments. In this case, identifying a medical instrument is possible, e.g., by means of the distance between the medical instrument's tip and at least one of the two localizers and, preferably, at least one of a medical instrument's angle or the length of that section of the sensor carrier that extends beyond the medical instrument's proximal end. For using the length of that section of the sensor carrier extends beyond the medical instrument's proximal end for identifying the medical instrument, in general, position and orientation of the medical instrument's proximal end as well as of the sensor carrier's proximal end have to be determined, e.g., with a position detection system. To this end, the sensor carrier can have a localizer arranged at the sensor carrier's distal end and another localizer arranged at a distance from the distal localizer towards the sensor carrier's proximal end. The position of the proximal localizer can be indicated, e.g., by a colour marking, such that for calibrating the medical can arrange the sensor carrier can be arranged in the medical instrument's lumen such that the colour marking is located at the proximal end of the medical instrument. In this case, the distance between the proximal localizer and the colour marking corresponds to the length of that section of the sensor carrier that extends beyond the medical instrument's proximal end and can be used for identifying the medical instrument.

With respect to surgical kit it is an advantage of the medical instrument identification setup that the medical instrument identification setup facilitates a fast and easy switching between medical instruments of the kit. Further, in a surgical kit the medical instruments of the kit can be designed so as to be easily discriminated and thus reliably recognized.

The calibration unit and the medical instrument identification unit can be components of data processing device, e.g., a computer, and can be implemented, e.g., by a processor, volatile and non-volatile computer memories and software.

The medical instrument identification unit can be configured for identifying a medical instrument with arranged sensor carrier by comparing a determined length of a lumen of the medical instrument with a plurality of lengths of different medical instruments comprised in a database. Preferably, the database contains the lengths of lumens at least of those medical instruments that are potentially being used during a navigated procedure.

In some embodiments of the medical instrument identification setup, the at least two localizers are arranged at a distance to each other close to a distal end of the sensor carrier. The sensor carrier can comprise at least a third localizer that is configured for providing a sensor signal representing position and orientation of the third localizer. Preferably, the third localizer is arranged at a distance from the first and second localizers towards the sensor carrier's proximal end. Preferably, the third localizer is arranged close to the proximal end of the sensor carrier. A sensor carrier with a third localizer being arranged close to the proximal end of the sensor carrier is particularly suitable for determining an angle of a medical instrument. Like the length of a medical instrument's lumen, the angle of medical instrument can be used for identifying the medical instrument. For example, a sensor carrier with a third localizer can be arranged in the lumen of a medical instrument such that a medical instrument's angle is located between the at least two localizers and the third localizer. From determined position and orientation of the third localizer and at least one of the at least two localizers, respectively, the medical instrument's angle can be calculated and used by the medical instrument identification unit for identifying the medical instrument with arranged sensor carrier. Advantageously, position and orientation of at least one of the localizers arranged in the medical instrument's distal end region and position and orientation of the third localizer can also be used for determining the sensor carrier's rotational orientation around the medical instrument's longitudinal axis, i.e., around the medical instrument's physical axis.

According to the invention a method for automatically identifying a medical instrument is proposed comprising the steps of
- providing a sensor carrier, configured to be removably arranged in a lumen of a medical instrument, the sensor carrier having at least two localizers, the localizers each being configured for providing a sensor signal representing position and orientation of the respective localizer,
- providing a calibration device, the position and orientation of which is known in the coordinate system of a position detection system,
- inserting the sensor carrier into a lumen of a medical instrument,
- determining position and orientation of the at least two localizers from provided sensor signals,
- calculating a distance between the calibration device and at least one of the two localizers based on the determined position and orientation of the at least two localizers to calibrate the medical instrument,
- determining the length of the medical instrument's lumen at least from the calculated distance, and
- using at least the determined length of the lumen for identifying the medical instrument.

The method can be conducted with the medical instrument identification setup according to the invention as described above.

Preferably, after insertion, the sensor carrier is arranged in the medical instrument's lumen such that the at least two localizers are arranged at a distance to each other along the longitudinal axis of the medical instrument.

In some variants of the method for automatically identifying a medical instrument, the method comprises the step of
- contacting a calibration device with a tip of the medical instrument.

It is particularly preferred that a distance between the calibration device and at least one of the two localizers is calculated based on position and orientation of at least one of the two localizers determined when the medical instrument's tip is in contact with the calibration device.

If the calibration device likewise is equipped with at least one localizer, position and orientation of the calibration device's localizer can also be determined with a position detection system in the position detection system's coordinate system. In particular, position and orientation of the calibration device's localizer can be determined with a position detection system and used by a calibration unit for calculating a distance between the calibration device and at least one of the two localizers, e.g., when the medical instrument's tip is in contact with the calibration device.

The medical instrument can be a medical screwdriver with a medical screw, e.g., a bone screw, attached at the screwdriver's distal end. The medical screwdriver's lumen with the arranged sensor carrier, preferably, extends from the proximal end of the screwdriver to the distal end of the attached screw. In particular, if the medical instrument is a medical screwdriver with a medical screw, the method for automatically identifying a medical instrument can comprise the step of determining the length of the medical screw.

The step of determining the length of the medical screw, preferably, comprises the substeps of
- contacting the calibration device with a tip of the medical screw, and, when in contact, determining position and orientation of the at least two localizers,
- calculating a distance between the calibration device and at least one of the two localizers based on the determined position and orientation, and
- determining the length of the screw at least from the calculated distance and using at least the determined length for identifying the medical screw.

Additionally, or alternatively, the method for automatically identifying a medical instrument according to the invention can comprise the steps of rotating the sensor carrier at least by 180°, preferably, by 360°, around its longitudinal axis, and, at the same time, determining position and orientation of the at least to localizers and using the determined positions and orientations to calculate a centering error and/or a positioning error and/or an angle error.

In particular, in variants of the method for automatically identifying a medical instrument in which a centering error and/or a positioning error and/or an angle error is calculated, preferably, the method can comprise the further step of compensating for the calculated centering error and/or positioning error and/or angle error when calculating the distance between the calibration device and at least one of the two localizers.

In particular, if the sensor carrier comprises at least a third localizer that is configured for providing a sensor signal representing position and orientation of the third localizer, the third localizer being arranged at a distance from the first and second localizers towards the sensor carrier's proximal end, the method for automatically identifying a medical instrument according to the invention can comprise the further steps of
- determining position and orientation of the third localizer,
- calculating an angle of a medical instrument from the position and orientation of the third localizer and position and orientation of at least one of the first and second localizers, and
- using the calculated angle for identifying the medical instrument.

Preferably, the medical instrument's lumen in which the sensor carrier is arranged is an off-centric lumen. Thereby, the medical instrument can comprise further lumens that can be used for different purposes, even, when the sensor carrier is arranged in the off-centric lumen.

### BRIEF DESCRIPTION OF THE DRAWINGS

In the following, preferred embodiments of the invention are described with reference to the figures. In the figures:
- Fig. 1: schematically shows a sensor carrier for connecting a medical instrument to a position detection system,
- Fig. 2:: schematically shows a medical instrument identification setup, comprising a sensor carrier and a calibration device;
- Fig. 3:: schematically shows a medical instrument with sensor carrier, the medical instrument contacting a calibration device,
- Fig. 4:: refers to the geometrical arrangement of the sensor carrier's localizers, medical instrument and the calibration device as schematically depicted in Fig. 3,
- Fig. 5:: schematically shows a screwdriver with an attached pedicle screw and a sensor carrier being arranged in the screwdriver's and the pedicle screw's lumen, the pedicle screw being in contact with a calibration device,
- Fig. 6:: refers to the geometrical arrangement of the sensor carrier's localizers, the screwdriver and the calibration device as schematically depicted in Fig. 5, and
- Fig. 7:: shows a flow diagram representing a method for automatically identifying a medical instrument.

### DETAILED DESCRIPTION

Figure 1 schematically shows a sensor carrier 100 for connecting a medical instrument to a position detection system. For connecting a medical instrument to a position detection system, the sensor carrier 100 can be inserted into a medical instrument's lumen. With the sensor carrier 100 being arranged in a medical instrument's lumen, a navigated procedure can be performed and the position of the medical instrument, e.g., inside a patient's body, can be displayed on a monitor. After having accomplished a navigated procedure, the sensor carrier can be removed from the medical instrument's lumen and inserted into the lumen of another medical instrument for connecting the other medical instrument to the position detection system.

The sensor carrier 100 has a distal end 102 and a proximal end 104. The sensor carrier 100 comprises three localizers 106, 108, 110 that are arranged distributed along the length of the sensor carrier 100. The localizers 106, 108, 110 each are configured for providing a sensor signal representing position and orientation of the respective localizer 106, 108, 110. For example, each of the localizers 106, 108, 110 can comprise at least one sensor coil, for connecting a medical instrument with arranged sensor carrier 100 to an electromagnetic position detection system.

Close to the sensor carrier's proximal end 102, a first localizer 106 of the three localizers is arranged. A second localizer 108 of the three localizers is arranged at a distance from the first localizer 106 towards the proximal end 104 of the sensor carrier 100. The sensor carrier 100 further comprises a third localizer 110 that is arranged at a distance from the first and second localizers 106, 108 towards the sensor carrier's proximal end 104. The third localizer 110 of the three localizers is optional and is not present in alternative embodiments of the sensor carrier 100.

The sensor carrier 100 having three localizers 106, 108, 110 is particularly suitable for identifying a medical instrument having an angle that is characteristic for the medical instrument. Preferably, for calculating an angle of a medical instrument, the sensor carrier 100 is arranged in the medical instrument's lumen such that the medial instrument's angle is located between the third localizer 110 and the first and second localizers 106, 108. From position and orientation determined for the localizers 106, 108, 110, respectively, the angle of the medical instrument can be calculated, e.g., with a calibration unit of a medical instrument identification setup, and used by the medical instrument identification setup's medical instrument identification unit for identifying the medical instrument with the sensor carrier 100 being arranged in its lumen.

The sensor carrier 100 further comprises a hypo tube 112 extending from the distal end 102 to the proximal end 104 of the sensor carrier 100 and enclosing the three localizers 106, 108, 110. The hypo tube 112 is configured to give mechanical stability to the sensor carrier 100 and to protect the localizers 106, 108, 110 from external influences.

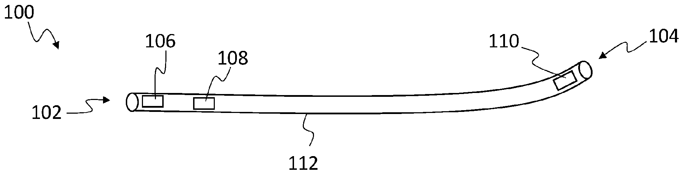

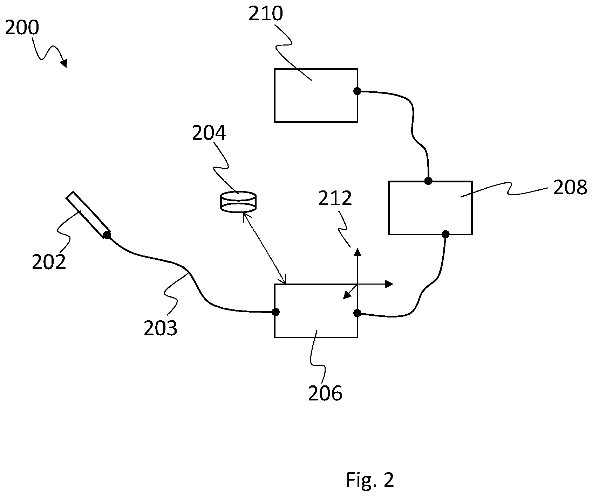

Figure 2 schematically shows a medical instrument identification setup 200 comprising a sensor carrier 202, a calibration device 204, a position detection system 206, a calibration unit 208, and a medical instrument identification unit 210.

The sensor carrier 202 can be configured the same way as the sensor carrier described with reference to figure 1. In particular, the sensor carrier 202 has at least two localizers (not shown) and can optionally have at least a third localizer that, preferably, is arranged close to the sensor carrier's proximal end. The localizers each are configured for providing a sensor signal representing position and orientation of the respective localizer. The sensor carrier 202 is configured to be removably arranged in a lumen of a medical instrument for connecting the medical instrument to the position detection system 206.

The sensor carrier 202 is connected to the position detection system 206 via a cable 203. In some embodiments of the medical instrument identification setup 200 provision is made of a sensor carrier that can be wirelessly connected to the position detection system 206. Via the cable 203, sensor signals provided by the localizers of the sensor carrier 202 can be transmitted to the position detection system 206. The position detection system 206 is configured for determining position and orientation of the localizers of the sensor carrier 202 in the position detection system's coordinate system 212 from received sensor signals.

For example, the position detection system 206 can be an electrometric position detection system having a field generator for generating an alternating electromagnetic field. For determining position and orientation of the sensor carrier's localizers, the localizers, preferably, comprise one or more sensor coils. When exposed to an alternating electromagnetic field, a voltage is induced in each of the coils that depends on position and orientation of the sensor coils in the alternating electromagnetic field. A sensor signal representing the induced voltage can be tapped from each of the sensor coils and transmitted to the position detection system for determining position and orientation of the sensor coils, respectively.

The calibration device 204 of the medical instrument identification setup 200 is configured such that its position and orientation in the position detection system's coordinate system 212 can be determined by the position detection system 206. For example, the calibration device 204 can be arranged at a position whose coordinate is known in the position detection system's coordinate system 212. It is also possible that the calibration device 204 likewise is equipped with one or more localizers, the position and orientation of which can be directly determined with the position detection system 206. The calibration device 204, preferably, is connected to the position detection system 206.

The position detection system 206 is connected to the calibration unit 208. The calibration unit 208 is configured for calibrating a medical instrument with the sensor carrier 202 arranged in its lumen by calculating a distance between the medical instrument's tip and at least one of the sensor carrier's localizers. The calibration unit 208 is configured for using the position and orientation of the calibration device 204 known in the position detection system's coordinate system 212 and position and orientation of at least one of the sensor carrier's localizers as determined by the position detection system 206. Preferably, the calibration unit is configured for determining whether or not the medical instrument's tip is in contact with the calibration device 204 and position and orientation of at least one of the sensor carrier's localizers that has been determined with the position detection system 206 at a moment of contact between the medical instrument's tip and the calibration device 204.

Since the distance between the medical instrument's tip and at least one of the sensor carrier's localizers is characteristic for the length of the lumen of a medical instrument, from a calculated distance the length of a lumen can be derived. Furthermore, since the length of a medical instrument's lumen is characteristic for the medical instrument itself, the determined length of a medical instrument's lumen can be used for automatically identifying the medical instrument having the sensor carrier 202 arranged in its lumen.

For identifying a medical instrument having the sensor carrier 202 arranged in its lumen, the medical instrument identification setup 200 comprises the medical instrument identification unit 210 that is connected to the calibration unit 208 for obtaining a calculated distance between a medical instrument's tip and at least one of the sensor carrier's localizers. The medical instrument identification unit 210 is configured for determining a length of the medical instrument's lumen at least from a distance between a medical instrument's tip and at least one of the sensor carrier's localizers as calculated by the calibration unit 208. The medical instrument identification unit 210 is configured for using the determined length of the medical instrument's lumen for identifying the medical instrument. The medical instrument identification unit 210 can be configured to alternatively or additionally to the lumen's length use a medical instrument's angle that has been calculated by the calibration unit 208 from position and orientation of at least two localizers between which the medical instrument's angle is located when the sensor carrier is arranged in the medical instrument's lumen. For identifying the medical instrument, the medical instrument identification unit 210 can be configured to compare the distance calculated by the calibration unit 208 and optionally also the medical instrument's angle to various lengths (and angles) of a plurality of medical instruments contained in a database that can be accessed by or that is part of the medical instrument identification unit 210.

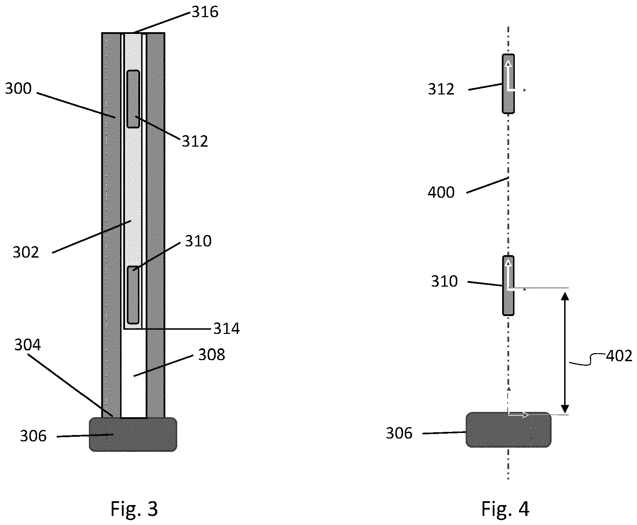

Figure 3 schematically shows a medical instrument 300 with sensor carrier 302. The medical instrument's tip 304 is in contact with a calibration device 306. The medical instrument 300 has a lumen 308 extending from the medical instrument's proximal end to its distal end. In the lumen 308 the sensor carrier 302 is arranged. The sensor carrier 302 comprises two localizers 310, 312, a first localizer 310 of the two localizers being arranged at the distal end 314 of the sensor carrier 302 and a second localizer 312 being arranged at a distance from the first localizer 310 towards the sensor carrier's proximal end 316.

Having two localizers arranged in the distal end region of the sensor carrier is of particularly advantage since this facilitates determining the trueness of the medical instrument's virtual longitudinal axis to the physical instrument's longitudinal axis. The virtual longitudinal axis can be extrapolated from the distal end of the sensor carrier to the distal end of the medical instrument to thus determine the position of the medical instrument's distal end. The medical instrument's virtual longitudinal axis can also be displayed in sectional images on a monitor. Displaying the virtual longitudinal axis in sectional images on a monitor can be of importance, e.g., when aiming with a medical instrument at an anatomical target from a large distance, e.g., from 5 to 15 cm. With an accurately displayed virtual longitudinal axis, e.g., of an access needle, a surgeon can assess position and orientation of the needle to reliably navigate the needle to a target location.

The virtual longitudinal axis can also be determined with one localizer implementing a 5 DOF sensor, only. However, using one 5 DOF sensorto determine the medical instrument's virtual longitudinal axis typically is subject to an angle error of the coil, an error introduced by the physical alignment inside the sensor carrier and an error introduced by the physical alignment of the sensor carrier inside the instrument. This can lead to angle errors between 4° and 5°.

Advantageously, when using two localizers each implementing a 5 DOF sensor, e.g., by using sensor coils, it is possible to reduce the angle error to only the position error of both sensor coils. Spacing the sensor coils 100 mm apart from each other, and having position errors of less than 1 mm, it is possible to reduce the angle error to less than 1° (tan(1/100)).

The length of the sensor carrier 302 is shorter than the length of the medical instrument 300 such that there exists a non-zero distance between the sensor carrier's distal end 314 and the medical instrument's tip 304. The distance is characteristic for the medical instrument and can be used for determining the length of the medical instrument's lumen 308. Since the length of the medical instrument's lumen 308 is characteristic for the medical instrument 300 itself, by means of the length of the medical instrument's lumen 308 the medical instrument 300 with arranged sensor carrier 302 can be identified.

The medical instrument 300 can be a medical instrument that has a lumen 308 provided, e.g., for advancing the medical instrument 300 over a guidewire. For example, the medical instrument 300 can be an access needle, a guiding rod, a working tube, tap, a balloon dilation device, or a screwdriver.

The sensor carrier 302 can be configured the same way as the sensor carrier described with reference to figure 1 or as the sensor carrier described with reference to figure 2. In particular, the sensor carrier 302 and the calibration device 306 can be elements of a medical instrument identification setup, e.g., of a medical instrument identification setup as described with reference to figure 2.

Figure 4 refers to the geometrical arrangement of the sensor carrier's localizers 310, 316, medical instrument and the calibration device 306 as described with reference to figure 3.

The two localizers 310, 316 are arranged at a distance to each other along the longitudinal axis 400 of the medical instrument 300.

For automatically identifying the medical instrument 300, the distance 402 between the calibration device 306 and the localizer 310 that is arranged close to the sensor carrier's distal end is calculated, e.g., by means of a calibration unit as described with reference to figure 2. For calculating the distance 402, preferably, position and orientation of the calibration device 306 and position and orientation of the localizer 310 as determined by a position detection system, e.g., of a medical instrument identification setup, can be used.

In particular, position and orientation of the localizer 310 can be determined when the medical instrument's tip is in contact with the calibration device 306 and used for calculating the distance 402. Advantageously, if the medical instrument's tip is in contact with the calibration device 306, the position of the medical instrument's tip can directly be associated with the position and orientation of the calibration device 306.

Position and orientation of the second localizer 312 can be used as a reference for performing a plausibility check on the calculated distance 402. Position and orientation of the first localizer 310 and the second localizer 312 can also be used for calculating a centering error and/or a positioning error and/or an angle error. The calculated centering error and/or positioning error and/or angle error can be compensated when calculating the distance 402 between the calibration device and the localizer 310. Thereby it is possible to calculate the distance 402 between the calibration device 306 and the localizer 310 with improved accuracy.

Having calculated the distance 402 between the calibration device 306 and the localizer 310, the distance 402 can be used for determining the length of the medical instrument's lumen 308 based on which the medical instrument 300 itself can be identified.

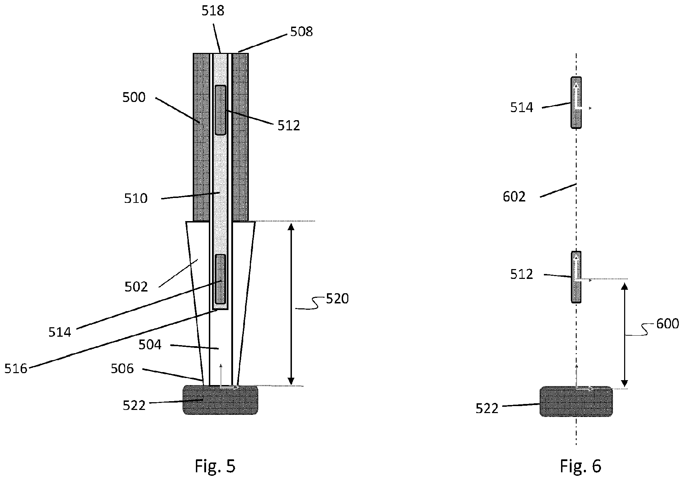

As in figure 3, in figure 5 a medical instrument with arranged sensor carrier is schematically shown, the medical instrument contacting a calibration device.

In figure 5, the medial instrument is a screwdriver 500 with an attached medical screw 502, e.g., a bone screw, preferably, a pedicle screw.

A lumen 504 extends from the screwdriver's proximal end 506 to the medical screw's distal end 508. In the lumen 508, the sensor carrier 510 is arranged. The sensor carrier 510 comprises two localizers 512, 514 wherein a first localizer 512 is arranged close to the sensor carrier's distal end 516 and the second localizer is arranged at a distance from the first localizer 516 towards the sensor carrier's proximal end 518. The sensor carrier 510 can be configured the same way as the sensor carrier as described with reference to figure 1 or with reference to figure 2.

The medical screw 502 has a length 520 that is characteristic for the medical screw 502. Thus, by determining the length of the screw, by means of the screw length the medical screw 502 itself can be identified.

With its distal end 506, the medical screw 502 is in contact with the calibration device 522 for calibrating the screwdriver 500, e.g., with a calibration unit of a medical instrument identification setup, e.g., as described with reference to figure 2. Calibrating the screwdriver comprises that a distance 600 between the calibration device 522 and the first localizer 512 is determined as depicted in figure 6.

Figure 6 refers to the geometrical arrangement of the sensor carrier's localizers 512, 514, the screwdriver and the calibration device 522 as schematically depicted in figure 5. The localizers 512, 514 are arranged along the screwdriver's longitudinal axis 602. In particular, position and orientation of the first localizer 512 are used for calculating the distance 600 to the calibration device which corresponds to the distance between the first localizer 512 and the medical screw's distal end 506.

The distance 600 is characteristic for the length of the medical screw 502. Therefore, the determined distance 600 can be used for automatically identifying the medical screw 502. For example, for identifying the medical screw 502 based on the determined distance 600, the length of the screwdriver 500 and the length of the sensor carrier 510 can be used. It can also be exploited that the positions of the localizers relative to each other are fixed. Preferably, the first localizer 512 is arranged close to the sensor carrier's distal end such that by determining the first localizer's position and orientation, position and orientation of the sensor carrier's distal end can be obtained.

The calibration device 522 and the sensor carrier 510 can be elements of a medical instrument identification setup, in particular, of a medical instrument identification setup as described with reference to figure 2.

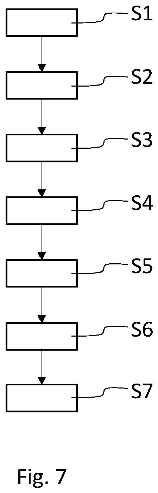

Figure 7 shows a flow diagram representing a method for automatically identifying a medical instrument.

Initially a sensor carrier is provided in step S1 which is configured to be removably arranged in a lumen of a medical instrument. The sensor carrier has at least two localizers. The localizers each are configured for providing a sensor signal representing position and orientation of the respective localizer. The sensor carrier can be a sensor carrier as described with reference to figure 1, or with reference to figure 2, or a sensor carrier as described with reference to figures 3 or 5.

A calibration device is provided in step S2, the position and orientation of which is known in the coordinate system of a position detection system. For example, the calibration device can be arranged at a position whose coordinate is known in the position detection system's coordinate system. It is also possible that the calibration device comprises one or more localizers that are configured for providing a sensor signal representing position and orientation of the calibration device.

The sensor carrier is inserted into a lumen of a medical instrument (step S3). The medical instrument having a lumen can, e.g. be a catheter, a Jamshidi needle, a tap, a screwdriver with an attached bone screw, e.g., a pedicle screw, that can be placed with the screwdriver into a patient's bone, or another cannulated medical instrument. After insertion, the sensor carrier is removably arranged in the medical instrument's lumen and can, thus, be removed after having accomplished a task with the medical instrument and used for connecting another medical instrument to a position detection system.

Position and orientation of the at least two localizers are determined from provided sensor signals in step S4. For example, the localizers can comprise one or more sensor coils the position of which can be determined with an electromagnetic position detection system having a field generator for generating an alternating electromagnetic field. When exposed to a generated electromagnetic field, a voltage is induced representing position and orientation of the sensor coils. From a tapped sensor signal representing the induced voltage, position and orientation of the sensor coils can be determined by the position detection system in the position detection system's coordinate system.

A distance between the calibration device and at least one of the two localizers is calculated in step S5 based on the determined position and orientation of the localizers to calibrate the medical instrument. Preferably, the distance is calculated based on position and orientation of the localizers determined when the medical instrument's tip is contacting a calibration device.

Afterwards, in step S6, the length of the medical instrument's lumen is determined at least from the calculated distance. In particular, the distance between the calibration device and at least one of the two localizers is characteristic for the length of the medical instrument's lumen and, thus, for the medical instrument itself.

Subsequently, in step S7, at least the determined length of the lumen is used for automatically identifying the medical instrument with the sensor carrier being arranged in its lumen. Additionally, also a medical instrument's angle and/or a length of that section of the sensor carrier that extends beyond the medical instrument's proximal end can be used for identifying the medical instrument. For identifying the medical instrument, the determined length of the lumen can be compared with entries of a database representing lengths of lumens of different medical instruments, e.g., of medical instruments of a surgical kit that is used during surgery.

If a medical instrument is automatically identified, e.g., by a medical instrument identification setup, settings, e.g., the mode of displaying the medical instrument on a monitor can be adapted accordingly.

The method can be conducted with a medical instrument identification setup, in particular, with a medical instrument identification setup as described with reference to figure 2.

## Claims

1. A medical instrument identification setup (200), comprising
- a sensor carrier (100, 202, 302, 510), configured to be removably arranged in a lumen (308, 504) of a medical instrument (300), the sensor carrier (1 00, 202, 302, 510) having at least two localizers (106, 108, 110, 310, 312; 512, 514), the localizers each being configured for providing a sensor signal representing position and orientation of the respective localizer,
- a position detection system (206), configured for determining position and orientation of the at least two localizers (106, 108, 110, 310, 312; 512, 514) in a position detection system's coordinate system (212) from received sensor signals,
- a calibration device (204, 306, 522), configured such that its position and orientation in the position detection system's coordinate system (212) can be determined by the position detection system (206),
- a calibration unit (208), configured for calibrating a medical instrument (300) with the sensor carrier (100, 202, 302, 510) arranged in its lumen (308, 504) by calculating a distance between the medical instrument's tip (304) and at least one of the two localizers (106, 108, 110, 310, 312; 512, 514) based on the position and orientation of the calibration device (204, 306, 522) and the position and orientation of at least one of the two localizers (106, 108, 110, 310, 312; 512, 514) determined by the position detection system (206), and
- a medical instrument identification unit (210), configured for determining a length of the medical instrument's lumen (308, 504) at least from the calculated distance and for using the determined length of the lumen (308, 504) for identifying the medical instrument (300).

2. The medical instrument identification setup (200) of claim 1, wherein the sensor carrier (100, 202, 302, 510) has a distal end (102, 314, 516) and a proximal end (104, 316, 518), a first one of the at least two localizers (106, 310, 514) being arranged at or at least close the distal end (102, 314, 516) of the sensor carrier (100, 202, 302, 510) and a second one of the localizers (106, 310 , 512) being arranged at a distance from the first localizer towards the proximal end (104, 316, 518) of the sensor carrier (100, 202, 302, 510).

3. The medical instrument identification setup (200) of claim 1 or 2, comprising at least a third localizer (110) that is configured for providing a sensor signal representing position and orientation of the third localizer (110), the third localizer (110) being arranged at a distance from the first and second localizers towards the sensor carrier's proximal end (104, 316, 518).

4. The medical instrument identification setup (200) of at least one of the preceding claims, wherein the calibration unit (208) is configured for determining a medical instrument's virtual longitudinal axis (400) based on the positions of the at least two localizers (106, 108, 310, 312; 512, 514).

5. The medical instrument identification setup (200) of at least one of the preceding claims, wherein the calibration unit (208) is configured for extrapolating a medical instrument's virtual longitudinal axis (400) from the distal end (102, 314, 516) of the arranged sensor carrier (100, 202, 302, 510) and for determining the position of a distal end of the medical instrument (300) in orderto calibrate the medical instrument (300).

6. The medical instrument identification setup (200) of at least one of the preceding claims, wherein the calibration unit (208) is configured for automatically detecting whether or not a medical instrument's tip (304) is in contact with the calibration device (204, 306, 522) by determining whether a virtual longitudinal axis (400) defined by the positions of the at least two localizers (106, 108, 110, 310, 312; 512, 514) intersects a known position of a calibration device (204, 306, 522) over a predefined period of time and for starting automatic calibration of the medical instrument (300) when the medical instrument's tip (304) is found to be in contact with the calibration device (204, 306, 522).

7. The medical instrument identification setup (200) of at least one of the preceding claims, the medical instrument identification unit (210) comprising a database that contains a plurality of lengths of different medical instruments (300), the medical instrument identification unit (210) being configured for identifying a medical instrument (300) with arranged sensor carrier (100, 202, 302, 510) by comparing a determined length of the lumen (308, 504) of the medical instrument (300) with the plurality of lengths of different medical instruments (300) contained in the database.

8. The medical instrument identification setup (200) of at least one of the preceding claims, wherein the medical instrument identification unit (210) is configured for identifying a medical instrument (300) based on a calculated angle of the medical instrument (300) and/or based on a length of a section of the sensor carrier (100, 202, 302, 510) that lies outside the medical instrument's lumen (308, 504) in which the sensor carrier (100, 202, 302, 510) is arranged.

9. A medical system comprising the medical instrument identification setup (200) according to at least one of the preceding claims and a surgical kit, the surgical kit comprising a plurality of medical instruments (300) having a lumen (308, 504) in which at least a part of the sensor carrier (100, 202, 302, 510) can be arranged.

10. A method for automatically identifying a medical instrument (300), the method comprising the steps of
- providing a sensor carrier (100, 202, 302, 510), configured to be removably arranged in a lumen (308, 504) of a medical instrument (300), the sensor carrier (100, 202, 302, 510) having at least two localizers (106, 108, 110, 310, 312; 512, 514), the localizers each being configured for providing a sensor signal representing position and orientation of the respective localizer,
- providing a calibration device (204, 306, 522), the position and orientation of which is known in the coordinate system (212) of a position detection system (206),
- inserting the sensor carrier (100, 202, 302, 510) into a lumen (308, 504) of a medical instrument (300),
- determining position and orientation of the at least two localizers (106, 108, 110, 310, 312; 512, 514) from provided sensor signals,
- calculating a distance between the calibration device (204, 306, 522) and at least one of the two localizers (106, 108, 110, 310, 312; 512, 514) based on the determined position and orientation to calibrate the medical instrument (300),
- determining the length of the medical instrument's lumen (308, 504) at least from the calculated distance, and
- using at least the determined length of the lumen (308, 504) for identifying the medical instrument (300).

11. The method of claim 10, wherein the medical instrument (300) is a medical screwdriver (500) with a medical screw attached at the screwdriver's distal end, wherein the medical screwdriver's lumen with the arranged sensor carrier (100, 202, 302, 510) extends from the proximal end (506) of the screwdriver (500) to the distal end (508) of the attached screw (502), the method comprising determining the length of the screw (502) by
- contacting the calibration device (204, 306, 522) with a tip of the medical screw, and, when in contact, determining position and orientation of the at least two localizers (106, 108, 110, 310, 312; 512, 514),
- calculating a distance between the calibration device (204, 306, 522) and at least one of the two localizers (106, 108, 310, 312; 512, 514) based on the respectively determined positions and orientations, and
- determining the length of the screw (502) at least from the calculated distance and using at least the determined length for identifying the medical screw (502).

12. The method of claim 10 or 11, comprising rotating the sensor carrier (100, 202, 302, 510) at least by 180° around its longitudinal axis (400), at the same time, determining position and orientation of the at least to localizers and using the determined positions and orientations to calculate a centering error and/or a positioning error and/or an angle error.

13. The method of claim 12, comprising compensating for the calculated centering error and/or positioning error and/or angle error when calculating the distance between the calibration device (204, 306, 522) and at least one of the two localizers (106, 108, 310, 312; 512, 514).

14. The method of at least one of claims 10 to 13, wherein the sensor carrier (100, 202, 302, 510) comprises at least a third localizer (110), configured for providing a sensor signal representing position and orientation of the third localizer (110), the third localizer (110) being arranged at a distance from the first and second localizers towards the sensor carrier's proximal end (104, 316, 518), the method comprising the steps of
- determining position and orientation of the at least three localizers,
- calculating an angle of a medical instrument (300) from the position and orientation of the third localizer (110) and position and orientation of at least one of the first and second localizers, and
- using the calculated angle for identifying the medical instrument (300).

15. The method of at least one of claims 10 to 14, comprising when having identified a medical instrument (300), automatically adapting the settings for displaying a digital representation of the medical instrument (300) on a monitor for the identified medical instrument (300).

## Patentansprüche

1. Anordnung zum Identifizieren eines medizinischen Instruments (200), umfassend
- einen Sensorträger (100, 202, 302, 510), der so konfiguriert ist, dass er herausnehmbar in einem Lumen (308, 504) eines medizinischen Instruments (300) angeordnet werden kann, wobei der Sensorträger (100, 202, 302, 510) mindestens zwei Lokalisatoren (106, 108, 110, 310, 312; 512, 514) aufweist, wobei die Lokalisatoren jeweils so konfiguriert sind, dass sie ein Sensorsignal bereitstellen, das die Position und Orientierung des jeweiligen Lokalisators wiedergibt,
- ein Positionserfassungssystem (206), das konfiguriert ist, Position und Orientierung der mindestens zwei Lokalisatoren (106, 108, 110, 310, 312; 512, 514) in einem Koordinatensystem (212) des Positionserfassungssystems aus empfangenen Sensorsignalen zu bestimmen,
- eine Kalibrierungsvorrichtung (204, 306, 522), die so konfiguriert ist, dass ihre Position und Orientierung im Koordinatensystem (212) des Positionserfassungssystems durch das Positionserfassungssystem (206) bestimmt werden kann,
- eine Kalibriereinheit (208), die zum Kalibrieren eines medizinischen Instruments (300) mit dem in seinem Lumen (308, 504) angeordneten Sensorträger (100, 202, 302, 510) durch Berechnen eines Abstands zwischen der Spitze (304) des medizinischen Instruments und mindestens einem der beiden Lokalisatoren (106, 108, 110, 310, 312; 512, 514) auf der Grundlage der Position und Orientierung der Kalibrierungsvorrichtung (204, 306, 522) und der Position Orientierung von mindestens einem der beiden Lokalisatoren (106, 108, 110, 310, 312; 512, 514), die durch das Positionserfassungssystem (206) bestimmt werden, und konfiguriert ist und
- eine Identifikationseinheit (210) für medizinische Instrumente, die konfiguriert ist, eine Länge des Lumens (308, 504) des medizinischen Instruments zumindest aus dem berechneten Abstand zu bestimmen und die bestimmte Länge des Lumens (308, 504) zum Identifizieren des medizinischen Instruments (300) zu verwenden.

2. Einrichtung (200) zum Identifizieren eines medizinischen Instruments nach Anspruch 1, wobei der Sensorträger (100, 202, 302, 510) ein distales Ende (102, 314, 516) und ein proximales Ende (104, 316, 518) aufweist, wobei ein erster der mindestens zwei Lokalisatoren (106, 310, 514) am oder zumindest nahe dem distalen Ende (102, 314, 516) des Sensorträgers (100, 202, 302, 510) angeordnet ist und ein zweiter der Lokalisatoren (106, 310, 512) in einem Abstand von dem ersten Lokalisator in Richtung des proximalen Endes (104, 316, 518) des Sensorträgers (100, 202, 302, 510) versetzt angeordnet ist.

3. Einrichtung (200) zum Identifizieren eines medizinischen Instruments nach Anspruch 1 oder 2, die mindestens einen dritten Lokalisator (110) umfasst, der so konfiguriert ist, dass er ein Sensorsignal bereitstellt, das die Position und Ausrichtung des dritten Lokalisators (110) wiedergibt, wobei der dritte Lokalisator (110) mit einem Abstand vom ersten und zweiten Lokalisator in Richtung des proximalen Endes (104, 316, 518) des Sensorträgers versetzt angeordnet ist.

4. Einrichtung (200) zum Identifizieren eines medizinischen Instruments nach mindestens einem der vorhergehenden Ansprüche, wobei die Kalibriereinheit (208) zum Bestimmen dervirtuellen Längsachse (400) eines medizinischen Instruments auf der Grundlage der Positionen der mindestens zwei Lokalisatoren (106, 108, 310, 312; 512, 514) konfiguriert ist.

5. Einrichtung (200) zum Identifizieren eines medizinischen Instruments nach mindestens einem der vorhergehenden Ansprüche, wobei die Kalibriereinheit (208) dazu ausgebildet ist, die virtuelle Längsachse (400) eines medizinischen Instruments aus dem distalen Ende (102, 314, 516) des darin angeordneten Sensorträgers (100, 202, 302, 510) zu extrapolieren und die Position eines distalen Endes des medizinischen Instruments (300) zu bestimmen, um das medizinische Instrument (300) zu kalibrieren.

6. Einrichtung (200) zum Identifizieren eines medizinischen Instruments nach mindestens einem der vorhergehenden Ansprüche, wobei die Kalibriereinheit (208) so konfiguriert ist, dass sie automatisch erkennt, ob die Spitze (304) eines medizinischen Instruments mit der Kalibrierungsvorrichtung (204, 306, 522) in Kontakt ist oder nicht, indem sie bestimmt, ob eine virtuelle Längsachse (400), die durch die Positionen der mindestens zwei Lokalisatoren (106, 108, 110, 310, 312; (106, 108, 110, 310, 312; 512, 514) definierte virtuelle Längsachse (400) eine bekannte Position einer Kalibrierungsvorrichtung (204, 306, 522) für die Dauer eines vordefinierten Zeitraum schneidet, und zum Starten der automatischen Kalibrierung des medizinischen Instruments (300), wenn festgestellt wird, dass die Spitze (304) des medizinischen Instruments mit der Kalibrierungsvorrichtung (204, 306, 522) in Kontakt ist.

7. Einrichtung (200) zum Identifizieren eines medizinischen Instruments nach mindestens einem der vorhergehenden Ansprüche, wobei die Identifikationseinheit (210) für medizinische Instrumente eine Datenbank umfasst, die eine Vielzahl von Längen unterschiedlicher medizinischer Instrumente (300) enthält, wobei die Identifikationseinheit (210) für medizinische Instrumente so konfiguriert ist, dass sie ein medizinisches Instrument (300) mit angeordnetem Sensorträger (100, 202, 302, 510) identifiziert, indem sie eine ermittelte Länge des Lumens (308, 504) des medizinischen Instruments (300) mit der in der Datenbank enthaltenen Vielzahl von Längen unterschiedlicher medizinischer Instrumente (300) vergleicht.

8. Einrichtung (200) zum Identifizieren eines medizinischen Instruments nach mindestens einem der vorhergehenden Ansprüche, wobei die Identifikationseinheit (210) für medizinische Instrumente ausgebildet ist ein medizinisches Instrument (300) auf der Basis eines berechneten Winkels des medizinischen Instruments (300) und/oder auf der Basis einer Länge eines Abschnitts des Sensorträgers (100, 202, 302, 510), der außerhalb des Lumens (308, 504) des medizinischen Instruments liegt, in dem der Sensorträger (100, 202, 302, 510) angeordnet ist, zu identifizieren.

9. Medizinisches System, umfassend die Einrichtung (200) zum Identifizieren eines medizinischen Instruments nach mindestens einem der vorhergehenden Ansprüche und ein chirurgisches Set, wobei das chirurgische Set eine Vielzahl von medizinischen Instrumenten (300) mit einem Lumen (308, 504) umfasst, in dem mindestens ein Teil des Sensorträgers (100, 202, 302, 510) angeordnet werden kann.

10. Verfahren zum automatischen Identifizieren eines medizinischen Instruments (300), wobei das Verfahren die folgenden Schritte umfasst
- Bereitstellen eines Sensorträgers (100, 202, 302, 510), der so konfiguriert ist, dass er herausnehmbar in einem Lumen (308, 504) eines medizinischen Instruments (300) angeordnet werden kann, wobei der Sensorträger (100, 202, 302, 510) mindestens zwei Lokalisatoren (106, 108, 110, 310, 312; 512, 514) aufweist, wobei die Lokalisatoren jeweils so konfiguriert sind, dass sie ein Sensorsignal bereitstellen, das die Position und Orientierung des jeweiligen Lokalisators darstellt,
- Bereitstellen einer Kalibrierungsvorrichtung (204, 306, 522), deren Position und Orientierung im Koordinatensystem (212) eines Positionserfassungssystems (206) bekannt ist,
- Einsetzen des Sensorträgers (100, 202, 302, 510) in ein Lumen (308, 504) eines medizinischen Instruments (300),
- Bestimmung von Position und Ausrichtung der mindestens zwei Lokalisatoren (106, 108, 110, 310, 312; 512, 514) aus bereitgestellten Sensorsignalen,
- Berechnen eines Abstands zwischen der Kalibrierungsvorrichtung (204, 306, 522) und mindestens einem der beiden Lokalisatoren (106, 108, 110, 310, 312; 512, 514) auf der Grundlage der ermittelten Position und Ausrichtung, um das medizinische Instrument (300) zu kalibrieren,
- Bestimmen der Länge des Lumens (308, 504) des medizinischen Instruments zumindest aus dem berechneten Abstand, und
- Verwenden zumindest der ermittelten Länge des Lumens (308, 504) zum Identifizieren des medizinischen Instruments (300).

11. Verfahren nach Anspruch 10, wobei das medizinische Instrument (300) ein medizinischer Schraubendreher (500) mit einer am distalen Ende des Schraubendrehers angebrachten medizinischen Schraube ist, wobei sich das Lumen des medizinischen Schraubendrehers mit dem darin angeordneten Sensorträger (100, 202, 302, 510) vom proximalen Ende (506) des Schraubendrehers (500) zum distalen Ende (508) der angebrachten Schraube (502) erstreckt, wobei das Verfahren das Bestimmen der Länge der Schraube (502) durch
- Berühren der Kalibrierungsvorrichtung (204, 306, 522) mit einer Spitze der medizinischen Schraube und, wenn der Kontakt besteht, Bestimmen der Position und Ausrichtung der mindestens zwei Lokalisatoren (106, 108, 110, 310, 312; 512, 514),
- Berechnen eines Abstands zwischen der Kalibrierungsvorrichtung (204, 306, 522) und mindestens einem der beiden Lokalisatoren (106, 108, 310, 312; 512, 514) auf der Grundlage der jeweils bestimmten Positionen und Ausrichtungen, und
- Bestimmen der Länge der Schraube (502) zumindest aus dem berechneten Abstand und Verwenden zumindest der ermittelten Länge zum Identifizieren der medizinischen Schraube (502),
umfasst.

12. Verfahren nach Anspruch 10 oder 11, bei dem der Sensorträger (100, 202, 302, 510) um mindestens 180° um seine Längsachse (400) gedreht wird, gleichzeitig die Position und Orientierung der mindestens zwei Lokalisatoren bestimmt wird und die so bestimmten Positionen und Orientierungen zur Berechnung eines Zentrierfehlers und/oder eines Positionsfehlers und/oder eines Winkelfehlers verwendet werden.

13. Verfahren nach Anspruch 12, umfassend das Kompensieren des berechneten Zentrierfehlers und/oder Positionierfehlers und/oder Winkelfehlers bei der Berechnung des Abstands zwischen der Kalibrierungsvorrichtung (204, 306, 522) und mindestens einem der beiden Lokalisatoren (106, 108, 310, 312; 512, 514).

14. Verfahren nach mindestens einem der Ansprüche 10 bis 13, wobei der Sensorträger (100, 202, 302, 510) mindestens einen dritten Lokalisator (110) umfasst, der so konfiguriert ist, dass er ein Sensorsignal bereitstellt, das die Position und Orientierung des dritten Lokalisators (110) darstellt, wobei der dritte Lokalisator (110) in einem Abstand von dem ersten und zweiten Lokalisator in Richtung des proximalen Endes (104, 316, 518) des Sensorträgers versetzt angeordnet ist, wobei das Verfahren die folgenden Schritte umfasst
- Bestimmen der Position und Ausrichtung der mindestens drei Lokalisatoren,
- Berechnen eines Winkels eines medizinischen Instruments (300) aus der Position und Ausrichtung des dritten Lokalisators (110) und der Position und Ausrichtung von mindestens einem der ersten und zweiten Lokalisatoren, und
- Verwenden des berechneten Winkels zum Identifizieren des medizinischen Instruments (300).

15. Verfahren nach mindestens einem der Ansprüche 10 bis 14, das, wenn ein medizinisches Instrument (300) identifiziert wurde, das automatische Anpassen der Einstellungen zur Anzeige einer digitalen Darstellung des medizinischen Instruments (300) auf einem Monitor für das identifizierte medizinische Instrument (300) umfasst.

## Revendications

1. Montage (200) d'identification d'un instrument médical, comprenant
- un support (100, 202, 302, 510) de capteur, configuré pour être disposé de manière amovible dans un lumen (308, 504) d'un instrument (300) médical, le support (100, 202, 302, 510) de capteur ayant au moins deux localiseurs (106, 108, 110, 310, 312 ; 512, 514), chaque localiseur étant configuré pour donner un signal de capteur représentant la position et l'orientation du localiseur respectif,
- un système (206) de détection de position, configuré pour déterminer la position et l'orientation des au moins deux localiseurs (106, 108, 110, 310, 312 ; 512, 514) dans un système (212) de coordonnées du système de détection de position à partir de signaux de capteur reçus,
- un dispositif (204, 306, 522) d'étalonnage, configuré de manière à ce que sa position et son orientation, dans le système (212) de coordonnées du système de détection de position, puissent être déterminées par le système (206) de détection de position,
- une unité (208) d'étalonnage, configurée pour étalonner un instrument (300) médical, alors que le support (100, 202, 302, 510) de capteur est disposé dans son lumen (308, 504), en calculant une distance entre le bout (304) de l'instrument médical et au moins l'un des deux localiseurs (106, 108, 110, 310, 312 ; 512, 514) sur la base de la position et de l'orientation du dispositif (204, 306, 522) d'étalonnage et de la position et de l'orientation d'au moins l'un des deux localiseurs (106, 108, 110, 310, 312 ; 512, 514) déterminées par le système (206) de détection de position, et
- une unité (210) d'identification d'un instrument médical, configurée pour déterminer une longueur du lumen (308, 504) de l'instrument médical au moins à partir de la distance calculée et pour utiliser la longueur déterminée du lumen (308, 504) pour identifier l'instrument (300) médical.

2. Montage (200) d'identification d'un instrument médical suivant la revendication 1, dans lequel le support (100, 202, 302, 510) de capteur a une extrémité (102, 314, 516) distale et une extrémité (104, 316, 518) proximale, un premier des au moins deux localiseurs (106, 310, 514) étant disposé à ou du moins près de l'extrémité (102, 314, 516) distale du support (100, 202, 302, 510) de capteur et un deuxième des localiseurs (106, 310, 512) étant disposé à une distance du premier localiseur en direction de l'extrémité (104, 316, 518) proximale du support (100, 202, 302, 510) de capteur.

3. Montage (200) d'identification d'un instrument médical suivant la revendication 1 ou 2, comprenant au moins un troisième localiseur (110), qui est configuré pour donner un signal de capteur représentant la position et l'orientation du troisième localiseur (110), le troisième localiseur (110) étant disposé à une distance des premier et deuxième localiseurs en direction de l'extrémité (104, 316, 518) proximale du support de capteur.

4. Montage (200) d'identification d'un instrument médical suivant au moins l'une des revendications précédentes, dans lequel l'unité (208) d'étalonnage est configurée pour déterminer un axe (400) longitudinal virtuel de l'instrument médical sur la base des positions des au moins deux localiseurs (106, 108, 310, 312 ; 512, 514).

5. Montage (200) d'identification d'un instrument médical suivant au moins l'une des revendications précédentes, dans lequel l'unité (208) d'étalonnage est configurée pour extrapoler un axe (400) longitudinal virtuel de l'instrument médical à partir de l'extrémité (102, 314, 516) distale du support (100, 202, 302, 510) de capteur disposé, et pour déterminer la position d'une extrémité distale de l'instrument (300) médical, afin d'étalonner l'instrument (300) médical.

6. Montage (200) d'identification d'un instrument médical suivant au moins l'une des revendications précédentes, dans lequel l'unité (208) d'étalonnage est configurée pour détecter automatiquement, si ou non, un bout (304) de l'instrument médical est en contact avec le dispositif (204, 306, 522) d'étalonnage, en déterminant si un axe (400) longitudinal virtuel, défini par les positions des au moins deux localiseurs (106, 108, 110, 310, 312 ; 512, 514), est en intersection avec une position connue d'un dispositif (204, 306, 522) d'étalonnage pendant un laps de temps défini à l'avance, et pour faire débuter un étalonnage automatique de l'instrument (300) médical, lorsqu'il est trouvé que le bout (304) de l'instrument médical est en contact avec le dispositif (204, 306, 522) d'étalonnage.

7. Montage (200) d'identification d'un instrument médical suivant au moins l'une des revendications précédentes, l'unité (210) d'identification d'un instrument médical comprenant une base de données, qui contient une pluralité de longueurs d'instruments (300) médicaux différents, l'unité (210) d'identification d'un instrument médical étant configurée pour identifier un instrument (300) médical, alors que le support (100, 202, 302, 510) de capteur y est disposé, en comparant une longueur déterminée du lumen (308, 504) de l'instrument (300) médical à la pluralité de longueurs d'instruments (300) médicaux différents contenus dans la base de données.

8. Montage (200) d'identification d'un instrument médical suivant au moins l'une des revendications précédentes, dans lequel l'unité (210) d'identification d'un instrument médical est configurée pour identifier un instrument (300) médical sur la base du calcul d'un angle de l'instrument (300) médical et/ou sur la base d'une longueur d'une partie du support (100, 202, 302, 510) de capteur, qui se trouve à l'extérieur du lumen (308, 504) de l'instrument médical, dans lequel le support (100, 202, 302, 510) de capteur est disposé.

9. Système médical comprenant le montage (200) d'identification d'un instrument médical suivant l'une des revendications précédentes et une trousse chirurgicale, la trousse chirurgicale comprenant une pluralité d'instruments (300) médicaux ayant un lumen (308, 504), dans lequel au moins une partie du support (100, 202, 302, 510) de capteur peut être disposée.

10. Procédé pour identifier automatiquement un instrument (300) médical, le procédé comprenant les stades :
- on se procure un support (100, 202, 302, 510) de capteur, configuré pour être disposé de manière amovible dans un lumen (308, 504) d'un instrument (300) médical, le support (100, 202, 302, 510) de capteur ayant au moins deux localiseurs (106, 108, 110, 310, 312 ; 512, 514), chaque localiseur étant configuré pour donner un signal de capteur représentant la position et l'orientation du localiseur respectif,
- on se procure un dispositif (204, 306, 522) d'étalonnage, dont la position et l'orientation est connue dans le système (212) de coordonnées d'un système (206) de détection de position,
- on insère le support (100, 202, 302, 510) de capteur dans un lumen (308, 504) d'un instrument (300) médical,
- on détermine la position et l'orientation des au moins deux localiseurs (106, 108, 110, 310, 312 ; 512, 514) à partir des signaux de capteur obtenus,
- on calcule une distance entre le dispositif (204, 306, 522) d'étalonnage et au moins l'un des deux localiseurs (106, 108, 110, 310, 312 ; 512, 514) sur la base de la position et de l'orientation déterminées pour étalonner l'instrument (300) médical,
- on détermine la longueur du lumen (308, 504) de l'instrument médical au moins à partir de la distance calculée, et
- on utilise au moins la longueur déterminée du lumen (308, 504) pour identifier l'instrument (300) médical.

11. Procédé suivant la revendication 10, dans lequel l'instrument (300) médical est un tournevis (500) ayant une vis médicale fixée à l'extrémité distale du tournevis, dans lequel le lumen du tournevis médical, alors que le support (100, 202, 302, 510) de capteur y est disposé, s'étend de l'extrémité (506) proximale du tournevis (500) à l'extrémité (508) distale de la vis (502) fixée, le procédé comprenant déterminer la longueur de la vis (502) en :
- mettant le dispositif (204, 306, 522) d'étalonnage en contact avec un bout de la vis médicale, et, alors qu'il y a contact, en déterminant la position et l'orientation des au moins deux localiseurs (106, 108, 110, 310, 312 ; 512, 514),
- calculant une distance entre le dispositif (204, 306, 522) d'étalonnage et au moins l'un des deux localiseurs (106, 108, 310, 312 ; 512, 514) sur la base des positions et orientations déterminées respectivement, et
- déterminant la longueur de la vis (502) au moins à partir de la distance calculée et en utilisant au moins la longueur déterminée pour identifier la vis (502) médicale.

12. Procédé suivant la revendication 10 ou 11, comprenant faire tourner le support (100, 202, 302, 510) de capteur d'au moins 180° autour de son axe (400) longitudinal, en même temps, déterminer la position et l'orientation des au moins deux localiseurs et utiliser les positions et les orientations déterminées pour calculer une erreur de centrage et/ou une erreur de positionnement et/ou une erreur d'angle.

13. Procédé suivant la revendication 12, comprenant compenser l'erreur calculée de centrage et/ou de positionnement et/ou d'angle, lors du calcul de la distance entre le dispositif (204, 306, 522) d'étalonnage et au moins des deux localiseurs (106, 108, 310, 312 ; 512, 514).

14. Procédé suivant au moins l'une des revendications 10 à 13, dans lequel le support (100, 202, 302, 510) de capteur comprend au moins un troisième localiseur (110), configuré pour donner un signal de capteur représentant la position et l'orientation du troisième localiseur (110), le troisième localiseur (110) étant disposé à une distance du premier et du deuxième localiseurs en direction de l'extrémité (104, 316, 518) proximale du support de capteur, le procédé comprenant les stades
- on détermine les positions et orientations des au moins trois localiseurs,
- on calcule un angle d'un instrument (300) médical, à partir de la position et de l'orientation du troisième localiseur (110) et de la position et de l'orientation d'au moins l'un des premier et deuxième localiseurs, et
- on utilise l'angle calculé pour identifier l'instrument (300) médical.

15. Procédé suivant au moins l'une des revendications 10 à 14, comprenant, lorsque l'on a identifié un instrument (300) médical, adapter automatiquement les réglages pour afficher une représentation numérique de l'instrument (300) médical sur un moniteur pour l'instrument (300) médical identifié.
